## Europäisches Patentamt

## European Patent Office

## Office européen des brevets

(19)

(11) Veröffentlichungsnummer: **0 046 897**
**B1**

(12) **EUROPÄISCHE PATENTSCHRIFT**

(45) Veröffentlichungstag der Patentschrift:
**19.10.83**

(51) Int. Cl.³: **C 07 D 213/06**

(21) Anmeldenummer: **81106248.8**

(22) Anmeldetag: **11.08.81**

(54) Verfahren zur Herstellung von Pyridin.

(30) Priorität: **16.08.80 DE 3031014**

(43) Veröffentlichungstag der Anmeldung:
**10.03.82 Patentblatt 82/10**

(45) Bekanntmachung des Hinweises auf die Patenterteilung:
**19.10.83 Patentblatt 83/42**

(84) Benannte Vertragsstaaten:
**BE CH DE FR GB IT LI NL**

(56) Entgegenhaltungen:
**DE-A-2 401 103**
**CHEMICAL ABSTRACTS, Band 91, 1979, Seite 572, Nr. 107871r Columbus, Ohio, U.S.A. L. FORNI et al.: »Pyridines by propylene ammoxidation over tellurium oxide/silica-alumina«**

(73) Patentinhaber: **HOECHST AKTIENGESELLSCHAFT, Postfach 80 03 20, D-6230 Frankfurt am Main 80 (DE)**

(72) Erfinder: **Bicker, Richard, Dr., Gebeschusstrasse 32, D-6230 Frankfurt am Main 80 (DE)**
Erfinder: **Erckel, Rüdiger, Dr., Staufenstrasse 16, D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Pyridin

Pyridin ist ein wichtiges Lösemittel sowie Ausgangs- oder Zwischenprodukt auf zahlreichen Sachgebieten, wie z. B. auf dem Pflanzenschutz- und Pharmasektor.

Zur Gewinnung und Herstellung von Pyridin und von Pyridin-Substitutionsprodukten (Pyridinbasen) sind verschiedene Wege und Verfahren bekannt. Eine instruktive Übersicht über den diesbezüglichen Stand der Technik zur Zeit des Jahres 1966 gibt z. B. der Artikel von K.-K. Moll »Über die Gewinnung von Pyridin und Alkylpyridinen« in der Zeitschrift Chem. Techn. 19, 528 bis 537 (1967).

Demnach erfolgte die Gewinnung von Pyridinbasen früher ausschließlich aus Kohleveredelungsprodukten. Außer daß auf diese Weise nur relativ geringe Mengen an Pyridinbasen zugänglich sind, bereitet die Auftrennung der hier stets im Gemisch erhaltenen Pyridinbasen erhebliche Schwierigkeiten; darüber hinaus sind die aus Kohle gewonnenen Pyridinbasen wegen ihres durchweg recht hohen Schwefelgehaltes für viele Zwecke ungeeignet.

Man hat daher schon frühzeitig versucht, rein synthetische Wege zur Herstellung von Pyridinbasen zu erschließen. Dabei ging man vor allem von aliphatischen Carbonylverbindungen — z. B. von Acrolein oder von Acetaldehyd + Formaldehyd — und Ammoniak aus. Die dabei erzielten Ausbeuten an Pyridinbasen sind jedoch relativ niedrig — sie liegen zwischen einigen Prozent bis etwa 20 bis 30% d. Th. —, und der Anteil an unsubstituiertem Pyridin darin ist ebenfalls niedrig. Reines Pyridin ist auf diese Weise nicht erhältlich.

Von Kohlenwasserstoffen wie z. B. von Cyclopentadien geht die sog. Ammozonolyse — d. i. die gemeinsame Einwirkung von Ozon und Ammoniak — aus. Dabei soll Pyridin in einer Ausbeute von 18% erhalten worden sein. Die Methode ist jedoch wegen des unangenehm handzuhabenden Ozons für die Übernahme in den technischen Maßstab kaum geeignet.

Auch die neueren Verfahren zur Herstellung von Pyridinbasen gehen hauptsächlich von Kohlenwasserstoffen aus. So werden nach dem in der GB-PS 1 134 163 (veröffentlicht 1968) beschriebenen 2-Stufen-Verfahren Propylen und/oder Butylene in der Dampfphase mit Sauerstoff oder einem sauerstoffhaltigen Gas bei Temperaturen zwischen 300 und 750° C zunächst katalytisch oxydiert und die dabei gebildeten Carbonylverbindungen dann mit Ammoniak bei Temperaturen bis zu 600° C zu Pyridinen kondensiert.

Als Oxydationskatalysatoren werden hierbei die für Oxydationen bekannten Katalysatoren, wie z. B. $V_2O_5$, V-B-P-O-Komplexe oder Te-Verbindungen verwendet; als Kondensationskatalysatoren dienen z. B. Kieselsäure, $Al_2O_3$, Alumosilikate, Kieselgur, Alundum oder Borphosphat.

Ausgehend von Propylen betragen die Ausbeuten an Pyridinbasen hier bis zu etwa 30% d. Th., bezogen auf umgesetztes Propylen, wobei etwa die Hälfte dieser Ausbeute auf Pyridin entfällt, (Rest: hauptsächlich Picoline). Reines Pyridin (ohne Homologe) fällt auch bei diesem Verfahren nicht an.

Man hat schließlich auch versucht, ausgehend von Kohlenwasserstoffen in einer einzigen Reaktionsstufe gleich zu Pyridin bzw. Pyridinbasen zu gelangen. Bei derartigen Verfahren werden die entsprechenden Kohlenwasserstoffe mit Sauerstoff oder einem sauerstoffhaltigen Gas (Luft!), Ammoniak und gegebenenfalls Wasserdampf an geeigneten Katalysatoren praktisch gleichzeitig oxydiert und kondensiert. Die unter solchen Bedingungen ablaufenden Verfahren sind unter der Bezeichnung »Ammoxydation« bekannt geworden.

Auf eine solche Ammoxydation bezieht sich die im Jahr 1973 erschienene JP-OS Sho-48-64020 der Firma Nissan (Japan). Danach werden N-haltige Verbindungen, insbesondere 1-Cyan-1,3-»pentadien« (womit wahrscheinlich 1-Cyan-1,3-butadien gemeint sein dürfte) und Pyridin, durch Ammoxydation von $C_5$-Kohlenwasserstoffen hergestellt. Als Katalysatoren sind die Oxyde von Wismuth, Vanadin, Molybdän und Phosphor angegeben, wobei das Atomverhältnis der Elemente Bi : V : Mo : P = (0-1) : (0-1) : (1-9) : >0,1 betragen soll. Trägersubstanzen für die Katalysatoren sind z. B. Tonerde oder Kolloidkieselsäure. Die Trägersubstanzen sollen die Ausbeute an 1-Cyan-1,3-»pentadien« gemäß der folgenden Reihe beeinflussen:

Aktivkohle < Kieselgel < Bimsstein < Carborundum < Alundum.

Mit sinkender Korngröße (und steigender Oberfläche) der Katalysatoren soll der Umsatz steigen.

Als Reaktionstemperaturen sind Temperaturen zwischen 450 und 550° C angegeben.

Die beste Pyridinausbeute beträgt bei Einsatz des Ausgangsproduktes 1,3-Pentadien 6%, bezogen auf umgesetztes 1,3-Pentadien.

Aus Cyclopentadien als Ausgangs-Kohlenwasserstoff wird nach der Tabelle am Ende der OS (mit dem Katalysator V-Mo-P-Oxyde auf Alundum als Träger) kein Pyridin erhalten.

Ein weiteres Ammoxydationsverfahren zur Herstellung von Pyridinbasen ist bekannt aus der im Jahr 1974 offengelegten JP-OS Sho 49-1569 der Firma Teijin (Japan). Ausgangs-Kohlenwasserstoffe für dieses Verfahren sind gesättigte oder ungesättigte Kohlenwasserstoffe mit mindestens 2 C-Atomen.

Katalysatoren sind saure Feststoffe, wobei folgende Materialien genannt sind: saure Tonmineralien (Bentonit, Kaolin), natürliche und synthetische kationische Zeolithe (vorzugsweise beladen mit Na-Ionen oder mit 2 bis 3wertigen Metallionen, insbesondere den 3wertigen Ionen der seltenen Erdmetalle), Kieselgel, Kieselsäure-Tonerde, Kieselsäure-MgO, Kieselsäure-Tonerde-MgO, Kieselsäure-Zirconerde, Sulfate (Nickelsulfat, Aluminiumsulfat), Phosphate (Calciumphosphat, Zinkphosphat),

Carbonate (CaCO₃) usw. In den Beispielen werden als Katalysatoren Kieselsäure-Tonerde und Zeolith verwendet.

Die Reaktionstemperaturen sollen zwischen 200 und 800°C, vorzugsweise zwischen 300 und 600°C liegen; als Druck für das Verfahren ist Normaldruck, jedoch auch Unter- und Überdruck angegeben.

Ausgehend von dem bevorzugten und auch in sämtlichen Beispielen verwendeten Kohlenwasserstoff Propylen werden geringe Ausbeuten an Pyridin — meist im Gemisch mit Picolinen — erhalten (etwa in Bsp. 1: 0,0090 Teile Pyridin/h ≙0,0096% Pyridin).

Unter den für das Verfahren in Frage kommenden Ausgangs-Kohlenwasserstoffen ist u.a. auch Cyclopentadien genannt. Wie eigene Versuche unter Einsatz eines Zeolith-Katalysators gezeigt haben, wird hier durch Ammoxydation von Cyclopentadien jedoch nur sehr wenig Pyridin erhalten.

Auch wenn der Zeolith-Katalysator etwa noch mit dem bekannten Oxydationskatalysator V₂O₅ dotiert wird, entsteht nach diesen eigenen Versuchen nur relativ wenig Pyridin.

Die Ammoxydation ebenfalls von gesättigten oder ungesättigten Kohlenwasserstoffen mit mindestens 2 C-Atomen an einem sauren Feststoff-Katalysator ist des weiteren bekannt aus der im Jahr 1975 offengelegten DE-OS 2 401 103 der Firma Teijin. Wesentliches Merkmal des hier verwendeten Katalysators ist der Gehalt an Tellur bzw. Tellurdioxid.

Das Tellurdioxyd kann aufgebracht oder vermischt sein mit sauren Tonmineralien (Bentonit, Kaolin), Oxyden wie SiO₂, Al₂O₃ V₂O₅, ZnO, zusammengesetzten Oxyden wie SiO₂ · Al₂O₃, SiO₂ · Al₂O₃ · ZrO₂, SiO₂ · MgO, Sulfaten (Nickelsulfat, Mangansulfat), Nitraten, Phosphaten, Carbonaten, Halogeniden.

Ein bevorzugter Katalysator enthält außer dem obligatorischen TeO₂ noch Sb₂O₃ und Platin.

Die Oberfläche der Katalysatoren soll mindestens 30 m²/g, vorzugsweise 50 m²/g betragen; aus den Beispielen sind Werte bis über 600 m²/g ersichtlich.

Als Reaktionstemperaturen sind Temperaturen zwischen 300 und 500°C, vorzugsweise zwischen 350 und 450°C angegeben.

Ausgehend von dem als Ausgangs-Kohlenwasserstoff bevorzugten Propylen werden Gemische von Pyridin und von dessen Homologen erhalten; die maximal erzielten Pyridinausbeuten liegen bei etwa 14%, bezogen auf umgesetztes Propylen, zusammen mit wechselnden Picolinmengen.

Unter den für das Verfahren in Frage kommenden Ausgangs-Kohlenwasserstoffen ist auch Cyclopentadien genannt; entsprechende Ausführungsbeispiele fehlen allerdings. Nach eigenen Versuchen wird aber durch Ammoxydation von Cyclopentadien an einem TeO₂-haltigen SiO₂-Katalysator kein Pyridin erhalten.

Auf der letztgenannten DE-OS 2 401 103 basiert der von den italienischen Autoren L. Forni und N. Stanga stammende Artikel in J. Catalysis 59, 148 bis 154 (1979), welcher sich mit einer näheren Untersuchung der Synthese von Pyridinbasen durch Ammoxydation von Propylen an einem TeO₂/SiO₂-Al₂O₃-Katalysator beschäftigt. In dem Artikel wird von den verschiedenen Methoden der katalytischen Ammoxydation von Olefinen die sich des TeO₂-Katalysators bedienende Methode als die aussichtsreichste (»most promising«) bezeichnet. Es werden sodann Untersuchungen an dem Katalysator TeO₂/SiO₂-Al₂O₃ ohne und mit anderen Oxyden (hier: Sb₂O₃, NiO, MnO₂) referiert. Dabei wurden Katalysatoren mit Oberflächen zwischen etwa 180 und 670 m²/g und Porenvolumina zwischen etwa 0,1 und 1,15 cm³/g verwendet und auch Messungen der Porenradien sowie der sauren Oberflächenzentren durchgeführt. Als Resultat der Untersuchungen ergab sich, daß der TeO₂-haltige SiO₂-Al₂O₃-Katalysator deswegen so gut für die Ammoxydation von Olefinen geeignet ist, weil er sowohl oxydierende als auch saure Zentren in optimaler Weise enthalten soll. An den oxydierenden Zentren (auf Basis TeO₂, vorzugsweise noch aktiviert durch Sb₂O₃) werden die Ausgangs-Kohlenwasserstoffe zu entsprechenden Carbonylverbindungen (wahrscheinlich Aldehyden) oxydiert, die dann an den sauren Zentren (auf Basis SiO₂-Al₂O₃) mit Ammoniak zu den entsprechenden Pyridinbasen kondensiert bzw. cyclisiert werden.

Trotz mancher Vorteile ist für die bekannten Synthesen von Pyridinbasen die relativ niedrige Ausbeute und die ebenfalls relativ niedrige Selektivität (fast immer Entstehen von schwer auftrennbaren Pyridinbasengemischen!) nachteilig. Wegen der steigenden Bedeutung des (unsubstituierten) Pyridins als Lösemittel und für synthetische Zwecke und wegen des dadurch bedingten steigenden Pyridin-Bedarfs bestand die Aufgabe, eine neue Pyridinsynthese zu entwickeln oder die bekannten Verfahren derart zu verbessern, daß Pyridin in höherer Ausbeute und Selektivität (möglichst ohne schwer abtrennbare Homologe!) entsteht.

Diese Aufgabe konnte erfindungsgemäß durch Ammoxydation von Cyclopentadien an einem bestimmten — Tellur-freien! — Katalysator bei einer relativ (verglichen mit den bekannten Verfahren) niedrigen Reaktionstemperatur gelöst werden.

Erfindungsgegenstand ist somit ein Verfahren zur Herstellung von Pyridin durch Ammoxydation von Cyclopentadien unter Verwendung eines Katalysators auf SiO₂ und/oder Al₂O₃-Basis mit großer Oberfläche und einem hohen Porenvolumen bei erhöhter Temperatur, das dadurch gekennzeichnet ist, daß man

a)  als Katalysator auf SiO₂- und/oder Al₂O₃-Basis Kieselgel, γ-Al₂O₃ und/oder ein Alumosilikat in tellurfreier Form, gegebenenfalls dotiert mit Oxyden von Übergangsmetallen und/oder von Gallium und/oder Indium mit Oberflächen von 75 bis 800 m²/g und Porenvolumina von 0,2 bis 2,0 cm³/g verwendet, und daß man
b) die Ammoxydation bei Temperaturen von 200 bis 400°C, vorzugsweise von 250 bis 350°C, durchführt.

Das Verfahren liefert Selektivitäten = auf umgesetztes Cyclopentadien bezogene Pyridinausbeuten bis zu etwa 45% und keine vom Pyridin schwer abtrennbaren Pyridin-Homologen.

Es war außerordentlich überraschend, daß durch die erfindungsgemäße Kombination an sich bekannter Elemente bei der an sich bekannten Ammoxydation von Olefinen ein gegenüber den bekannten Olefin-Ammoxydationen ganz erheblich verbessertes Ergebnis erzielt wird, weil aufgrund der Tabelle am Ende der JP-OS Sho 48-64020 sowie der eigenen Versuche der Nacharbeitung des Verfahrens der JP-OS Sho 49-1569 sowie der DE-OS 2 401 103 mit den dortigen Katalysatortypen und Cyclopentadien als Ausgangs-Olefin nicht zu erwarten war, daß in dieser Richtung überhaupt noch ein brauchbares — geschweige denn fortschrittliches — Resultat erzielbar ist.

Es war insbesondere im Hinblick auf die Aussage in J. Catalysis 59, 148 bis 154 (1979) über die entscheidende und für einen optimalen Verlauf der Ammoxydation wesentliche Bedeutung des $TeO_2$, nicht zu erwarten, daß mit einem tellurfreien Katalysator sogar noch eine bessere Pyridinausbeute und -selektivität erhalten wird.

Als Oxydationsmittel für die erfindungsgemäße Ammoxydation kann sowohl reiner Sauerstoff als auch ein sauerstoffhaltiges Gas eingesetzt werden. Bevorzugt ist die Verwendung von Luft. Das Molverhältnis zwischen Sauerstoff und dem Cyclopentadien in dem dem Reaktionsraum zugeführten Ausgangsgemisch liegt zweckmäßig im Bereich zwischen 0,5 bis 75 zu 1, wobei ein Verhältnis im Bereich von 1 bis 20 : 1 bevorzugt ist.

Auch das Molverhältnis zwischen Ammoniak und Olefin in dem Ausgangsgemisch kann in weiten Grenzen schwanken. Zweckmäßig liegt das Molverhältnis bei 0,5 bis 50 zu 1, vorzugsweise bei 10 bis 30 : 1.

Wasserdampf ist als Verdünnungsmittel bei dem erfindungsgemäßen Verfahren nicht unbedingt notwendig; seine Anwesenheit ist jedoch zweckmäßig. Wenn in Gegenwart von Wasserdampf gearbeitet wird, soll das molare Verhältnis von Wasserdampf zu Cyclopentadien im Ausgangsgemisch im Bereich von 10 bis 100 : 1, vorzugsweise von 15 bis 65 : 1 liegen.

Die Katalysatoren für das erfindungsgemäße Verfahren sind Kieselgel, $\gamma$-$Al_2O_3$ und/oder Aluminiumsilikat(e) in tellurfreier Form. Die Katalysatoren können als solche oder gegebenenfalls — und bevorzugt — in mit Oxyden von Übergangsmetallen dotierter Form verwendet werden. Bevorzugtes Übergangsmetalloxyd für diesen Zweck ist $V_2O_5$. Auch die Oxyde der Hauptgruppenelemente Gallium und Indium zeigen eine katalytische Wirksamkeit. Die Menge dieser Dotierungsmittel kann zwischen 0,1 und 40 Gew.-%, bezogen auf den Gesamtkatalysator, betragen; bevorzugt sind Mengen von 0,5 bis 10 Gew.-% Dotierungsmittel.

Neben den Dotierungsmitteln können die Katalysatoren auch noch andere übliche Zusätze wie Alkalioxyde, Alkalisulfate, Erdalkalioxyde und Edelmetalle der Platingruppe — insbesondere Platin selbst — enthalten. Die Menge der Nichtedelmetall-Zusatzstoffe kann in der gleichen Größenordnung wie die Menge der Dotierungsmittel liegen; die Edelmetallmengen sind geringer: sie liegen zweckmäßig zwischen nur 0,05 und 3%, vorzugsweise zwischen nur 0,1 und 1,0%.

Die gegebenenfalls dotierten und ebenfalls gegebenenfalls noch mit anderen üblichen Zusatzstoffen versehenen Katalysatoren sollen Oberflächen zwischen 75 und 800 m²/g (bestimmt nach der Brunauer-Emmet-Teller = BET-Methode) sowie Porenvolumina zwischen 0,2 und 2,0 cm³/g besitzen.

Beispielhafte Katalysatoren sind in der folgenden Tabelle aufgeführt:

0 046 897

| Katalysator | Übergangsmetall-oxyd | Weitere Zusatz-stoffe | BET-Ober-fläche | Poren-volumen |
|---|---|---|---|---|
| Kieselgel | + 5,5% $V_2O_5$ | | 302 m²/g | 0,567 cm³/g |
| Kieselgel | 5,5% $V_2O_5$ | | 570 m²/g | 0,36 cm³/g |
| Kieselgel | 10% $V_2O_5$ | +10% $K_2SO_4$ | 682 m²/g | 0,667 cm³/g |
| Kieselgel | 12,5% $V_2O_5$ | +12,5% $K_2SO_4$ | 158 m²/g | 0,191 cm³/g |
| Kieselgel | 12,6% $V_2O_5$ | + 0,8% $P_2O_5$ | 173 m²/g | 0,344 cm³/g |
| Kieselgel | 10% $V_2O_5$ | +10% $K_2SO_4$ | 226 m²/g | 0,424 cm³/g |
| Kieselgel | 10% $V_2O_5$ | +30% $K_2SO_4$ | 149 m²/g | 0,343 cm³/g |
| Kieselgel | — | + 5,6% $In_2O_3$ | 283 m²/g | 0,523 cm³/g |
| Kieselgel | — | + 3,1% $SnO_2$ | 283 m²/g | 0,532 cm³/g |
| Kieselgel | 1,6% ZnO | | 283 m²/g | 0,523 cm³/g |
| Kieselgel | 11,0% $V_2O_5$ | + 1,1% $Rb_2O$ | 283 m²/g | 0,523 cm³/g |
| Kieselgel | 2,3% $V_2O_5$ | | 283 m²/g | 0,523 cm³/g |
| Kieselgel | 2,0% $V_2O_5$ | | 680 m²/g | 0,45 cm³/g |
| Kieselgel | 10%/2% $MnO_2$ | + 1% $K_2SO_4$ | 283 m²/g | 0,523 cm³/g |
| Kieselgel/0,6%/$\gamma$-$Al_2O_3$ | 7,7% $V_2O_5$ | 7,7% $K_2SO_4$ | 265 m²/g | 0,538 cm³/g |
| $\gamma$-$Al_2O_3$ | 10% $V_2O_5$ | | 78 m²/g | 0,33 cm³/g |
| $\gamma$-$Al_2O_3$ | 8,9% $V_2O_5$ | | 250 m²/g | 0,55 cm³/g |
| $\gamma$-$Al_2O_3$ | 12,8% $V_2O_5$ | | 100 m²/g | 0,55 cm³/g |
| $\gamma$-$Al_2O_3$ | 10%/1% $MoO_3$ | | 78 m²/g | 0,33 cm³/g |
| $\gamma$-$Al_2O_3$ | 10%/1% $Fe_2O_3$ | | 78 m²/g | 0,33 cm³/g |
| $\gamma$-$Al_2O_3$ | 10% | 1% $SnO_2$ | 78 m²/g | 0,33 cm³/g |
| $\gamma$-$Al_2O_3$ | 10%/1% ZnO | | 78 m²/g | 0,33 cm³/g |
| $\gamma$-$Al_2O_3$ | 5% | 0,5% $P_2O_5$ | 100 m²/g | 0,55 cm³/g |
| $\gamma$-$Al_2O_3$ | 10%/1% $TiO_2$ | | 250 m²/g | 0,55 cm³/g |
| $\gamma$-$Al_2O_3$ | 20% $V_2O_5$ | 2% $K_2TiF_6$ | 300 m²/g | 0,47 cm³/g |
| Al-Silikat (74,5% $SiO_2$ + 25% $Al_2O_3$) | 10% $V_2O_5$ | 10% $K_2SO_4$ | 100 m²/g | 0,28 cm³/g |
| Al-Silikat (86,5% $SiO_2$ + 13% $Al_2O_3$) | 10% $V_2O_5$ | 10% $K_2SO_4$ | 375 m²/g | 0,4 cm³/g |

Die Herstellung der Katalysatoren kann nach irgendeinem der bekannten Verfahren zur Herstellung von Katalysatoren hergestellt werden. Eine beispielhafte Herstellungsweise besteht darin, daß vorgefertigtes Kieselgel, $\gamma$-$Al_2O_3$ und/oder Aluminiumsilikat mit einer entsprechenden Metallsalzlösung imprägniert wird. Nach der Trocknung wird der imprägnierte Katalysator im Luftstrom bei etwa

5

200 bis 600° C, vorzugsweise bei etwa 350 bis 450° C calciniert und die Metallsalze so in die Oxyde überführt.

Als einzusetzende Metallsalze werden vorzugsweise Nitrate, Chloride, Oxalate, Acetate, Tartrate und Acetylacetonate, gelöst in Wasser oder Alkohol oder auch in anderen Lösungsmitteln, wie z. B. Methylenchlorid, Toluol oder Dimethylformamid, verwendet.

Es ist auch möglich, den fertigen Katalysator vor Gebrauch zu reduzieren, wobei als Reduktionsmittel z. B. Ammoniak, Olefin, Wasserstoff oder Hydrazinhydrat verwendbar sind.

Wenn sich im Laufe der erfindungsgemäßen Ammoxydationsreaktion auf dem Katalysator Polymerabscheidungen bilden, kann eine Regeneration bzw. Reaktivierung des Katalysators durch eine Behandlung mit Luft etwa bei 300 bis 500° C erfolgen. Die Polymerabscheidungen werden dabei abgebrannt.

Die Reaktion wird bei einer Temperatur im Bereich von 200 bis 400° C durchgeführt, wobei der bevorzugte Temperaturbereich zwischen 250 und 350° C liegt.

Die Reaktion wird vorzugsweise bei atmosphärischem oder geringfügig höherem Druck durchgeführt.

Die scheinbare Kontaktzeit ist nicht kritisch. Es können scheinbare Kontaktzeiten im Bereich von 0,1 bis 10 sec angewandt werden. Im allgemeinen ist jedoch eine Kontaktzeit im Bereich von 0,1 bis 5 sec bevorzugt.

Zur Durchführung des erfindungsgemäßen Verfahrens kann jede Apparatur eingesetzt werden, die zur Durchführung von Oxydationsreaktionen in der Dampfphase zum Einsatz kommen. Das Verfahren kann entweder kontinuierlich oder diskontinuierlich durchgeführt werden, wobei im Festbett oder in einer Wirbelschicht gearbeitet werden kann.

Es empfiehlt sich, das Ausgangsgasgemisch vorzuheizen.

Das Katalysatorbett kann ein Festbett unter Anwendung grober Katalysatorteilchen sein, wie unregelmäßige Formkörper, Strangpreßlinge, Kugeln, Pellets oder Tabletten, es kann jedoch auch mit einem in Wirbelschicht angeordneten Katalysator durchgeführt werden.

Der den Reaktor verlassende Gasstrom enthält im wesentlichen Pyridin, Kohlenoxyde, Acetonitril und Blausäure. Diese Umsetzungsprodukte können aus dem Gasstrom nach an sich bekannten Verfahren getrennt werden. Eines dieser Verfahren besteht z. B. darin, das den Reaktor verlassende Gas mit kaltem Wasser zu waschen, wobei das Pyridin und das Acetonitril im wesentlichen aus dem Gasstrom entfernt werden. Zur besseren Absorption des Pyridins kann das Wasser auch angesäuert sein, wobei in diesem Fall auch gleichzeitig das nichtumgesetzte Ammoniak neutralisiert und Ammoniumcarbonatbildung aus nichtumgesetztem Ammoniak und Kohlendioxyd vermieden wird.

Die gebildete Blausäure verbleibt ebenfalls im Waschwasser. Der die Wasserwäsche verlassende Gasstrom enthält im wesentlichen nicht umgesetztes Olefin, Kohlendioxyd, Stickstoff und Sauerstoff und kann bei geringerem Umsatz nach Abtrennung des Kohlendioxyds dem Reaktor wieder zugeführt werden bzw. wird bei weitgehend vollständigem Umsatz verworfen.

Die endgültige Gewinnung des Pyridins geschieht nach allgemein üblichen Methoden.

Das nicht umgesetzte Olefin kann auch aus dem die Wasserwäsche verlassenden Gasstrom durch Extraktion mit einem unpolaren Lösungsmittel, wie z. B. Methylenchlorid, entfernt und so zurückgewonnen werden.

Die Erfindung ermöglicht — ausgehend von einem einfach und gut zugänglichen Ausgangsmaterial (Cyclopentadien) — die Herstellung von Pyridin in Selektivitäten bis zu etwa 45% d. Th., und — was besonders hervorzuheben ist — ohne daß dabei Picoline und andere homologe Pyridinbasen mitgebildet werden. Die sonst (bei den meisten bekannten Verfahrensweisen) notwendige schwierige Abtrennung des Pyridins aus einem Pyridinbasen-Gemisch entfällt hier also. Die Erfindung stellt daher einen erheblichen Fortschritt auf diesem Gebiet dar; denn bei den vergleichbaren bekannten Verfahren sind die Pyridinausbeuten und -selektivitäten teilweise erheblich niedriger.

Eine detaillierte Beschreibung des erfindungsgemäßen Verfahrens und der Katalysatorherstellung erfolgt in Verbindung mit den Ausführungsbeispielen. Die in den Beispielen angegebenen Ausbeuten, Umsätze und Selektivitäten sind wie folgt definiert:

$$\text{Ausbeute:} \quad \frac{\text{Menge gebildetes Pyridin}}{\text{Menge max. erwartetes Pyridin}} \cdot 100$$

$$\text{Umsatz:} \quad \frac{\text{Menge einges. Cyclopentadien} - \text{Menge zurückgew. Cyclopentadien}}{\text{Menge einges. Cyclopentadien}} \cdot 100$$

$$\text{Selektivität:} \quad \frac{\text{Ausbeute}}{\text{Umsatz}} \cdot 100$$

Der in den Beispielen 1—9 eingesetzte Reaktor war ein Standardreaktor aus VA-Stahl mit einem Durchmesser von 2 cm. Der Reaktor besitzt ein inneres axiales Rohr als Temperaturmeßseele, bei dem die Temperatur in der Katalysatorschüttung an mehreren Punkten mit einem Thermoelement gemessen werden kann. Beheizt wird das Reaktorrohr durch eine Salzschmelze. Die Gase wurden

6

## 0 046 897

mittels Rotametern in den Reaktor eingemessen, die $H_2O$-Dosierung erfolgte mit einer Schlauchpumpe, das Cyclopentadien wurde aus einer gekühlten Waschflasche durch einen Stickstoffstrom in den Reaktor gefördert.

Die den Reaktor verlassenden Produktgase werden durch ein System von Fallen und Waschflaschen geleitet und schließlich gaschromatographisch analysiert.

Die in den folgenden Beispielen im Festbettreaktor eingesetzten Katalysatoren wurden nach der Imprägniermethode hergestellt.

Nach den Erfindungsbeispielen (A) folgen dann noch einige Vergleichsbeispiele (B), welche zeigen, daß die Ammoxydation von Cyclopentadien an verschiedenen Katalysatoren des Standes der Technik kein oder nur sehr wenig Pyridin liefert.


### A) Erfindungsbeispiele

#### Beispiel 1

Ein Katalysator mit der Zusammensetzung 10 Gew.-% $V_2O_5$, 30 Gew.-% $K_2SO_4$ auf Kieselgel B (BET-Oberfläche: 283 $m^2/g$, Porenvolumen: 0,523 $cm^3/g$) wurde wie folgt hergestellt:

Mit einer Lösung aus 25,8 g $NH_4VO_3$, 51,6 g Oxalsäure und 60 g $K_2SO_4$ in 400 ml $H_2O$ wurden 120 g Kieselgel B viermal imprägniert. Nach jedem Imprägnierschritt trocknet man 16 h bei 110°C.

Die Aktivierung erfolgt durch Kalzinierung 6 h lang bei 400°C in einem Luftstrom (8 l/h Luft). Die Katalysatorteilchengröße betrug durchschnittlich 2 – 3 mm.

46 g des obigen Katalysators wurden in den Reaktor eingefüllt und bei 300°C 2,6 g/h Cyclopentadien, 10 l/h $NH_3$, 17,5 l/h Luft, 18 g/h $H_2O$ und 6 l/h $N_2$ durch den Reaktor geleitet. Versuchszeit: 4 h.

Ausbeute an Pyridin: 12,5%; Umsatz: 100%; Selektivität: 12,5%.


#### Beispiel 2

Der eingesetzte Katalysator entspricht jenem in Beispiel 1. 50,3 g dieses Katalysators werden in den Reaktor eingefüllt und bei 300°C 2,6 g/h Cyclopentadien, 10 l/h $NH_3$, 17,5 l/h Luft, 18 g/h $H_2O$ und 6 l/h $N_2$ durch den Reaktor geleitet.

Nach einer Laufzeit von einer halben Stunde wird jeweils mit 6 l/h Luft regeneriert, wobei das Fallensystem am Reaktorausgang abgeschaltet wird. Mit dieser Fahrweise wird die Standzeit des Kontaktes wesentlich verlängert.

Ausbeute an Pyridin: 11,7%; Umsatz: 94,1%; Selektivität: 12,5%.


#### Beispiel 3

Ein Katalysator mit ca. 5,5% $V_2O_5$ auf Kieselgel A (BET-Oberfläche: 560 $m^2/g$; Porenvolumen: 0,29 $cm^3/g$) wurde wie folgt hergestellt:

Mit einer Lösung aus 12,9 g $NH_4VO_3$ und 25,8 g Oxalsäure in 60 ml $H_2O$ werden 60 g Kieselgel A imprägniert.

Aus der Lösungsmengeaufnahme errechnet sich ein Gehalt von 5,5% $V_2O_5$ im fertigen Kontakt.

Die Trocknung und Aktivierung erfolgt wie in dem Beispiel 1 beschrieben.

16,0 g dieses Katalysators werden in den Reaktor gefüllt und bei 300°C 0,79 g/h Cyclopentadien, 10 g/h $NH_3$, 18 g/h $H_2O$ und 3,1 l/h $N_2$ durch den Reaktor geleitet.

Versuchsdauer: 4 h.

Ausbeute an Pyridin: 9,7%; Umsatz 67,3%; Selektivität: 14,5%.


#### Beispiel 4

Ein Katalysator mit ca. 5,5% $V_2O_5$ auf Kieselgel B wird analog Beispiel 3 hergestellt: 8,2 g dieses Katalysators werden in den Reaktor gefüllt und bei 300°C 0,79 g/h Cyclopentadien, 5,0 l/h $NH_3$, 17,5 l/h Luft, 3,3 l/h $N_2$ und 18 g/h $H_2O$ durch den Reaktor geleitet. Versuchsdauer: 4 h.

Ausbeute an Pyridin: 8,2%, Umsatz: 66,4%; Selektivität: 12,3%.


#### Beispiel 5

Der Katalysator aus Beispiel 4 wird mit 50%iger Hydrazinhydratlösung versetzt und im $N_2$-Strom getrocknet.

7

10,3 g dieses Katalysators werden in den Reaktor gefüllt und bei 300°C 0,7 g/h Cyclopentadien, 5,0 l/h NH$_3$, 17,5 l/h Luft, 3,3 l/h N$_2$ und 18 g/h H$_2$O durch den Reaktor geleitet. Versuchsdauer: 4 h.
Ausbeute an Pyridin: 13,0%; Umsatz: 75,2%; Selektivität: 17,3%.

## Beispiel 6

Ein Katalysator mit 1,57% V$_2$O$_5$ auf Kieselgel E wurde wie folgt hergestellt:
Mit einer Lösung aus 1,29 g NH$_4$VO$_3$ und 2,58 g Oxalsäure in 50 ml H$_2$O werden 49 g Kieselgel E zweimal imprägniert. Trocknung und Aktivierung analog Beispiel 1.
15 g dieses Katalysators werden in den Reaktor gefüllt und bei 300°C 0,6 g/h Cyclopentadien, 5,0 l/h NH$_3$, 17,5 l/h Luft, 18 g/h H$_2$O und 3,0 l/h N$_2$ durch den Reaktor geleitet. Versuchsdauer: 4 h.
Ausbeute an Pyridin: 20,6%; Umsatz 71,9%; Selektivität: 28,7%.

## Beispiel 7

Ein Katalysator mit 1,6% ZnO auf Kieselgel B wurde wie folgt hergestellt: Mit einer Lösung aus 2,61 g Zinknitrat [Zn(NO$_3$)$_2$ · 4 H$_2$O] in 80 ml H$_2$O wurden 47,5 g Kieselgel B zweimal imprägniert. Trocknung und Kalzinierung wie in Beispiel 1.
8,8 g dieses Katalysators wurden in den Reaktor gefüllt und bei 300°C 0,7 g/h Cyclopentadien, 5,0 l/h NH$_3$, 17,5 l/h Luft, 18 g/h H$_2$O und 3,2 l/h N$_2$ durch den Reaktor geleitet. Versuchsdauer: 4 h.
Ausbeute an Pyridin: 3,1%; Umsatz: 42,4%; Selektivität: 7,3%.

## Beispiel 8

Ein Katalysator mit 2,6% V$_2$O$_5$ und 0,1% Pt auf Kieselgel B wurde wie folgt hergestellt:
Mit einer Lösung aus 23,5 g NH$_4$VO$_3$ und 47,0 g Oxalsäure in 700 ml H$_2$O wurden 475 g Kieselgel B zweimal imprägniert.
Trocknung und Kalzinierung analog Beispiel 1. Dies ergibt einen Katalysator mit ca. 2,6% V$_2$O$_5$ auf Kieselgel B. 50 g dieses Katalysators werden mit einer Lösung von 66,6 mg PtCl$_2$ in 50 ml 5 n-HCl imprägniert. Trocknung und Kalzinierung analog Beispiel 1.
9,0 g dieses Katalysators werden in den Reaktor gefüllt und bei 300°C 0,6 g/h Cyclopentadien, 5,0 l/h NH$_3$, 17,5 l/h Luft, 18 g/h H$_2$O und 3,0 l/h N$_2$ durch den Reaktor geleitet. Versuchsdauer: 4 h.
Ausbeute an Pyridin: 11,4%; Umsatz: 59,1%; Selektivität: 19,3%.
Ohne Platin, d. h. bei Einsatz des Vorkatalysators mit 2,6% V$_2$O$_5$ auf Kieselgel B erzielt man folgendes Ergebnis:
Ausbeute an Pyridin: 8,5%; Umsatz: 70,0%; Selektivität: 12,1%.

## Beispiel 9

Ein Katalysator mit der Zusammensetzung 2,3% V$_2$O$_5$ auf Kieselgel B (BET-Oberfläche: 283 m$^2$/g, Porenvolumen: 0,523 cm$^3$/g) wurde wie folgt hergestellt:
2,65 g Vanadylacetylacetonat wurden in 60 ml Methylenchlorid gelöst und 80 g Kieselgel B mit dieser Lösung imprägniert. Das überschüssige Methylenchlorid wird am Rotavapor abgezogen. Der so erhaltene Katalysator wird wie im Beispiel 1 beschrieben getrocknet und kalziniert.
7,17 g obigen Katalysators wurden in den Reaktor eingefüllt und bei 300°C 0,6 g/h Cyclopentadien, 5,0 l/h NH$_3$, 17,5 l/h Luft, 18 g/h H$_2$O und 1,1 l/h N$_2$ durch den Reaktor geleitet. Versuchszeit: 4 h.
Ausbeute an Pyridin: 6,6%; Umsatz: 47,7%; Selektivität: 15,4%.

## Beispiel 10

Ein Katalysator mit 20% V$_2$O$_5$ auf $\gamma$-Al$_2$O$_3$ als Träger (BET-Oberfläche 80 m$^2$/g; Porenvolumen: 0,45 cm$^3$/g) wurde wie folgt hergestellt:
Mit einer Lösung aus 12,9 g Ammoniumvanadat (NH$_4$VO$_3$) und 25,8 g Oxalsäure in 40 ml H$_2$O werden 40 g $\gamma$-Al$_2$O$_3$-Kugeln zweimal imprägniert. Nach jedem Imprägnierungsschritt trocknet man 16 h bei 110°C. Die Aktivierung erfolgt wie im Beispiel 1 beschrieben.
12,4 g obigen Katalysators werden in den Reaktor gefüllt und bei 300°C 0,5 g/h Cyclopentadien, 5 l/h NH$_3$, 17,5 l/h Luft, 3,0 l/h N$_2$ und 18 g/h H$_2$O durch den Reaktor geleitet. Versuchsdauer: 4 h.
Ausbeute an Pyridin: 5,3%; Umsatz: 98,8%; Selektivität: 5,4%.

**0 046 897**

## Beispiel 11

Als Katalysator wird undotiertes Kieselgel E (BET-Oberfläche: 667 m$^2$/g, Porenvolumen: 0,31 cm$^3$g) eingesetzt. Das Gel wird mit Wasser behandelt, worauf die Kieselgelkugeln, die Durchmesser von 3−5 mm besitzen, unter starker Wärmeentwicklung zerspringen und schließlich Durchmesser um 1 mm aufweisen. Das Gel wird dann 8 h bei 150°C getrocknet und so eingesetzt.

49,9 g obigen Gels werden in den Reaktor gefüllt und bei 300°C 0,4 g/h Cyclopentadien, 5,0 l/h NH$_3$, 17,5 l/h Luft, 18 g/h H$_2$O und 0,5 l/h N$_2$ durch den Reaktor geleitet. Versuchszeit 4 h.

Ausbeute an Pyridin: 20,6%; Umsatz: 98,4%; Selektivität: 20,8%.

## Beispiel 12

16,9 g des in Beispiel 6 beschriebenen Katalysators werden in den Reaktor gefüllt und bei 300°C 0,3 g/h Cyclopentadien, 5,0 l/h NH$_3$, 17,5 l/h Luft und 20 l/h N$_2$ durch den Reaktor geleitet. Versuchsdauer: 5 h.

Ausbeute an Pyridin: 37,0%; Umsatz: 82,7%; Selektivität: 44,7%.

## B) Vergleichsbeispiele

### Vergleichsbeispiel 1
(Verwendung von Zeolith- = Molekularsiebkatalysatoren entspr. JP-OS Sho-49-1569)

a) undotierter Molekularsiebkatalysator
Katalysator: Molekularsieb 13 x (Fa. Union Carbide Corp./USA)
46,2 g Molekularsieb 13 x werden in den Reaktor eingefüllt und bei 300°C 9,4 g/h Cyclopentadien, 10 l/h NH$_3$, 17,5 l/h Luft, 16 g/h H$_2$O und 11 l/h N$_2$ durch den Reaktor geleitet. Versuchszeit: 4 h, Ausbeute an Pyridin: 0%,

b) mit V$_2$O$_5$ dotierter Molekularsiebkatalysator
b1) 7,2% V$_2$O$_5$ auf Molsieb 13 x (Fa. Union Carbide), Pyridin-Ausbeute: 1,22%,
b2) 5% V$_2$O$_5$ auf Molsieb K 306 (Fa. Süd-Chemie),
Pyridin-Ausbeute: 3,05%,
b3) 5% V$_2$O$_5$ auf Molsieb AW 500 (Fa. Linde),
Pyridin-Ausbeute: 1,3%,
b4) 10% V$_2$O$_5$, 10% K$_2$SO$_4$ auf Molsieb KA-120 (Fa. Süd-Chemie),
Pyridin-Ausbeute: 2,4%; Selektivität: 4,6%.

### Vergleichsbeispiel 2
(Verwendung eines TeO$_2$-haltigen SiO$_2$-Katalysators entspr. DE-OS 2 401 103)

Ein Tellurdioxidhaltiger Katalysator mit ca. 3,2% TeO$_2$ auf Kieselgel B (BET-Oberfläche 283 m$^2$/g, Porenvolumen: 0,523 cm$^3$/g) wurde wie folgt hergestellt:

Mit einer Lösung aus 1,6 g TeO$_2$ in 100 ml halbkonzentrierter HNO$_3$ (ca. 30 Gew.-%) werden 47,5 g Kieselgel B zweimal imprägniert. Nach jeder Imprägnierung trocknet man 16 h lang bei 110°C. Die Kalzinierung erfolgt bei 400°C 6 h lang im Luftstrom. 8,1 g obigen Katalysators werden in den Reaktor eingefüllt und bei 300°C 0,9 g/h Cyclopentadien, 5 l/h NH$_3$, 17,5 l/h Luft, 15 g/h H$_2$O und 3,0 l/h N$_2$ durch den Reaktor geleitet. Versuchszeit: 4 h.

Ausbeute an Pyridin: 0%.

### Vergleichsbeispiel 3
(Verwendung eines TeO$_2$-haltigen SiO$_2$-Katalysators entspr. DE-OS 2 401 103)

Ein tellurhaltiger Katalysator mit ca. 10% TeO$_2$ auf Kieselgel E (BET-Oberfläche: 667 m$^2$/g, Porenvolumen: 0,523 cm$^3$/g) wurde wie folgt hergestellt:

Mit einer Lösung aus 5,5 g TeO$_2$ in 150 ml HNO$_3$ konz. werden 45 g Kieselgel E dreimal imprägniert. Nach jeder Imprägnierung trocknet man 16 h lang bei 110°C. Die Kalzinierung erfolgt bei 550°C 4 h lang im Muffelofen ohne Luftstrom.

12,9 g obigen Katalysators werden in den Reaktor eingefüllt und bei 420°C 5,8 g/h Cyclopentadien = 0,088 Mol/h, 2,3 l/h NH$_3$, 15 l/h Luft, 20 g/h H$_2$O und 8,0 l/h N$_2$ durch den Reaktor geleitet. Die Bedingungen entsprechen denen des Beispiels 1 (Tabelle 1, Ansatz 1a) in der DE-OS 2 401 103, nur daß Cyclopentadien anstatt Propylen umgesetzt wird. Versuchsdauer: 4 h.

Ausbeute an Pyridin: 0%.

9

**0 046 897**

**Patentansprüche**

1. Verfahren zur Herstellung von Pyridin durch Ammoxidation von Cyclopentadien unter Verwendung eines Katalysators auf $SiO_2$- und/oder $Al_2O_3$-Basis mit großer Oberfläche und einem hohen Porenvolumen bei erhöhter Temperatur, dadurch gekennzeichnet, daß man

a) als Katalysator auf $SiO_2$- und/oder $Al_2O_3$-Basis Kieselgel, $\gamma$-$Al_2O_3$ und/oder Aluminiumsilikat(e) in tellurfreier Form, gegebenenfalls dotiert mit Oxyden von Übergangsmetallen und/oder von Gallium und/oder Indium, mit einer Oberfläche zwischen 75 und 800 $m^2/g$ und einem Porenvolumen zwischen 0,2 und 2 $cm^3/g$ verwendet, und daß man

b) die Ammoxidation bei Temperaturen von 200 bis 400° C durchführt.

2. Verfahren nach Anspruch 1, dadurch gekennzeichnet, daß man die Ammoxidation bei Temperaturen von 250 bis 350° C durchführt.

3. Verfahren nach Ansprüchen 1 bis 2, dadurch gekennzeichnet, daß der Katalysator mit $V_2O_5$ dotiert ist.

4. Verfahren nach den Ansprüchen 1 bis 3, dadurch gekennzeichnet, daß der Katalysator mit 0,5 bis 10 Gew.-% Dotierungsmittel dotiert ist.

5. Verfahren nach den Ansprüchen 1 bis 4, dadurch gekennzeichnet, daß der Katalysator noch 0,5 bis 10 Gew.-% Alkali- und/oder Erdalkaliverbindungen und/oder 0,05 bis 3 Gew.-% Edelmetalle der Platingruppe enthält.

**Claims**

1. Process for the preparation of pyridine by ammoxidation of cyclopentadiene using a catalyst based on $SiO_2$ and/or $Al_2O_3$ with a large surface area and a large pore volume at elevated temperature, characterized in

a) using as catalyst based on $SiO_2$ and/or $Al_2O_3$ silica gel, $\gamma$-$Al_2O_3$ and/or aluminium silicate(s) in tellurium-free form, optionally doped with oxides of transition metals and/or of gallium and/or indium, having a surface area of between 75 and 800 $m^2/g$ and a pore volume of between 0,2 and 2 $cm^3/g$ and

b) carrying out the ammoxidation at temperatures of from 200 to 400° C.

2. Process as claimed in claim 1, characterized in carrying out the ammoxidation at temperatures from 250 to 350° C.

3. Process as claimed in claims 1 to 2 characterized in using the catalyst doped with $V_2O_5$.

4. Process as claimed in claims 1 to 3, characterized in using the catalyst doped with 0,5 to 10% by weight of the doping agent.

5. Process as claimed in claims 1 to 4, characterized in using the catalyst also containing 0,5 to 10% by weight of alkali- and/or earthalkalimetal compounds and/or 0,05 to 3% by weight of noble metals of the platinum group.

**Revendications**

1. Procédé de préparation de la pyridine par ammoxydation du cyclopentadiène à l'aide d'un catalyseur à base de $SiO_2$ et/ou d'$Al_2O_3$ ayant une surface importante et un volume de pores élevé, à température élevée, procédé caractérisé en ce que:

a) on utilise, comme catalyseur à base de $SiO_2$ et/ou d'$Al_2O_3$, du gel de silice, de l'alumine $\gamma$ et/ou un ou plusieurs silicates d'aluminium sous forme exempte de tellure, le cas échéant dopés avec des oxydes de métaux de transition et/ou de gallium et/ou d'indium, ayant une surface comprise entre 75 et 800 $m^2/g$, et un volume de pores compris entre 0,2 et 2 $cm^3/g$, et

b) on effectue l'ammoxydation à une température de 200 à 400° C.

2. Procédé suivant la revendication 1, caractérisé en ce qu'on effectue l'ammoxydation à une température de 250 à 350° C.

3. Procédé suivant l'une des revendications 1 et 2, caractérisé en ce que le catalyseur est dopé par $V_2O_5$.

4. Procédé suivant l'une quelconque des revendications 1 à 3, caractérisé en ce que le catalyseur est dopé avec 0,5 à 10% en poids d'agent dopant.

5. Procédé suivant l'une quelconque des revendications 1 à 4, caractérisé en ce que le catalyseur contient en outre de 0,5 à 10% en poids de composés alcalins et/ou alcalino-terreux et/ou de 0,05 à 3% en poids de métaux nobles du groupe du platine.

10